Europäisches Patentamt

European Patent Office

Office européen des brevets

(11) Veröffentlichungsnummer: **0 453 967 A1**

## (12) EUROPÄISCHE PATENTANMELDUNG

(21) Anmeldenummer: **91106266.9**

(22) Anmeldetag: **18.04.91**

(51) Int. Cl.5: **C07B 41/04**, C07C 43/11,
C07C 41/03

(30) Priorität: **21.04.90 DE 4012725**

(43) Veröffentlichungstag der Anmeldung:
**30.10.91 Patentblatt 91/44**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**W-6230 Frankfurt am Main 80(DE)**

(72) Erfinder: **Wimmer, Ignaz**
**Burger Strasse 26**
**W-8261 Winhöring(DE)**

(54) Verfahren zur Herstellung von Alkoxylaten mit enger Homologenverteilung.

(57) Beim beschriebenen Verfahren erfolgt die Alkoxylierung von aktive H-Atome enthaltenden Verbindungen mit Antimonpentahalogeniden als Katalysator in der Weise, daß zunächst eine Katalysator-Vormischung bereitet wird, indem man Antimonpentahalogenid in einer Teilmenge der zur Alkoxylierung vorgesehenen Verbindung auflöst unter Einhaltung einer Temperatur von maximal 60 °C und einer Konzentration an Antimonpentahalogenid von maximal 30 Mol-%, die bereitete Katalysator-Vormischung oder ein Teil davon als Katalysatorkomponente mit der restlichen Menge der zu alkoxylierenden Verbindung vereinigt wird und die so erhaltene Startmischung mit dem Alkylenoxid umgesetzt wird.

EP 0 453 967 A1

Rank Xerox (UK) Business Services

Die Erfindung betrifft ein Verfahren zur Herstellung von Alkoxylaten mit einer engen Homologenverteilung durch Umsetzung von mindestens ein aktives H-Atom enthaltenden Verbindungen mit einem Alkylenoxid unter Verwendung von Antimonpentahalogeniden als Katalysator.

Derartige Alkoxylierungsverfahren sind schon seit langem bekannt, zum Beispiel aus der britischen Patentschrift 796,508 und der US-Patentschrift 3,359,331. Beim Verfahren der britischen Patentschrift werden die zu alkoxylierende Verbindung und das Antimonpentahalogenid als solches in einem Reaktionsgefäß vorgelegt, worauf die Mischung bei Reaktionstemperatur mit Alkylenoxid umgesetzt wird. Das erhaltene Alkoxylat weist zwar eine relativ enge Homologenverteilung auf, das sind sogenannte "narrow-range"- Alkoxylate, es läßt aber insbesondere bezüglich Farbe und Gehalt an Nebenprodukten zu wünschen übrig. Ein Nachteil des bekannten Verfahrens ist ferner, daß infolge des Einsatzes des Antimonpentahalogenids in Substanz dessen Korrosivität und problematische Handhabung in besonders hohem Ausmaß auftreten. Auf diese Nachteile wird in der genannten US-Patentschrift 3,359,331 hingewiesen (Spalte 1, Zeilen 27 bis 54). Zu ihrer Vermeidung wird die Alkoxylierung in der Weise durchgeführt, daß man zunächst eine Vormischung bereitet, indem man nur einen Teil der vorgesehenen Mengen an Katalysator, alkoxylierender Verbindung und Alkylenoxid in einem Reaktiongefäß aus Glas, Keramik und dergleichen umsetzt. Diese Vormischung (Samen-Produkt) wird dann in einem Reaktionsgefäß aus Metall mit den restlichen Mengen an Katalysator, alkoxylierender Verbindung und Alkylenoxid vereinigt, worauf die Mischung weiter alkoxyliert wird. Es liegt auf der Hand, daß dieses Verfahren umständlich ist und zu Alkoxylaten führt, die eine breitere Homologenverteilung haben, als mit Antimonpentahalogenid als Katalysator zu erwarten wäre.

Die Aufgabe der Erfindung besteht darin, ein Verfahren zur Alkoxylierung von aktive H-Atome enthaltenden Verbindungen unter Verwendung von Antimonpentahalogeniden zur Verfügung zu stellen, das die genannten Nachteile nicht aufweist, andererseits aber die vorteilhafte Eigenschaft von Antimonpentahalogeniden nützt, nämlich "narrow-range"-Alkoxylate zu bilden. Das neue Verfahren soll also eine problemlosere Handhabung von Antimonpentahalogeniden im Rahmen der Alkoxylierung bieten und Alkoxylate in hoher Ausbeute liefern, die nicht nur eine enge Homologenverteilung, sondern auch eine gute Farbqualität aufweisen.

Das erfindungsgemäße Verfahren ist dadurch gekennzeichnet, daß

a) mit einer Teilmenge der zur Alkoxylierung vorgesehenen Verbindung eine maximal 30 Mol-%, bezogen auf die eingesetzte Verbindungsmenge, Antimonpentahalogenid enthaltende Katalysator-Vormischung bereitet wird, durch Auflösen von Antimonpentahalogenid in der eingesetzten Verbindungsmenge unter Einhaltung einer Temperatur von maximal 60 °C und der genannten Konzentration an Antimonpentahalogenid von maximal 30 Mol-%, und

b) diese Vormischung als Katalysatorkomponente eingesetzt wird, indem man die gesamte bereitete Vormischung oder einen Teil davon mit der restlichen Menge der zu alkoxylierenden Verbindung vereinigt, und

c) die so erhaltene Startmischung mit dem Alkylenoxid umgesetzt wird, mit der Maßgabe, daß in der Startmischung eine Antimonpentahalogenidmenge von 0,05 bis 1 Mol-% vorliegt, vorzugsweise 0,2 bis 0,6 Mol-%, bezogen auf die zu alkoxylierende Verbindungsmenge.

Das erfindungsgemäße Verfahren unterscheidet sich vom Stand der Technik gemäß genannter britischer Patentschrift 796,508 dadurch, daß das Antimonpentahalogenid nicht als solches eingesetzt wird, sondern in Form einer bestimmten Lösung in der zu alkoxylierenden Verbindung, und vom Stand der Technik gemäß genannter US-Patentschrift 3,359,331 dadurch, daß bei der Bereitung der Katalysator-Vormischung Alkylenoxid nicht beteiligt ist.

Beim erfindungsgemäßen Verfahren werden also in einem ersten Schritt [Schritt a)] ein Teil der zur Alkoxylierung vorgegebenen Verbindungsmenge und das in Substanz vorliegende und als Katalysator einzusetzende Antimonpentahalogenid zu einer Lösung vermischt. Beim Vermischen sollen 60 °C nicht überschritten und ferner die Konzentration des Antimonpentahalogenids in der Lösung auf maximal 30 Mol-% gebracht werden. Bei einer Temperatur von mehr als 60 °C im Verlauf der exothermen Lösungsreaktion besteht die Gefahr von Farbbildung. Wenn auch niedrige Temperaturen nicht kritisch sind, wird man den Auflösungsvorgang aus Zweckmäßigkeitsgründen unter 20 °C nicht durchführen. Die bevorzugte Auflösungstemperatur liegt demnach bei 20 bis 60 °C. Wenn die Konzentration des Antimonpentahalogenids in der Lösung auf über 30 Mol-% gebracht wird, besteht ebenfalls die Gefahr der Farbbildung. Niedrige Katalysator-Konzentrationen sind andererseits nicht zweckmäßig. Aus diesen Gründen wird man die in Rede stehende Konzentration auf einen Wert von 5 bis 30 Mol-% einstellen, vorzugsweise auf 10 bis 20 Mol-%, Molprozente bezogen auf die eingesetzte Menge (Teilmenge) an zu alkoxylierender Verbindung. Was die Menge an bereiteter Katalysator-Vormischung betrifft, so ist diese für das erfindungsgemäße Verfahren nicht kritisch. In der Regel wird man eine solche Menge an Vormischung bereiten, daß damit nicht nur eine, sondern mehrere (zeitlich mehr oder weniger weit auseinanderliegende) Alkoxylierungen durchgeführt werden können, wobei jedesmal ein Teil der Katalysator-Vormischung eingesetzt wird, und zwar soviel, daß in der Startmischung die angegebene Antimonpentahalogenid-Konzentration vorhanden ist. Ausgehend vom Fall, daß eine bestimmte Menge an Verbindung (Produkt) vorliegt, die alkoxyliert werden soll, wird man etwa 1 bis 20 Gew.-% davon, vorzugsweise 1 bis 10 Gew.-%, zur Bereitung der Katalysator-Vormischung einsetzen. Das Vermischen der beiden Komponenten wird vorzugsweise so durchgeführt, daß die Verbindung vorgelegt und das Antimonpentahalogenid portionsweise oder kontinuierlich, bevorzugt kontinuierlich, dazugegeben wird unter Rühren und Einhalten der angegebenen Temperatur, gegebenenfalls durch Kühlen. Die erhaltene, an Antimonpentahalogenid relativ konzentrierte Lösung stellt die angestrebte Katalysator-Vormischung (Katalysatorkomponente) dar.

Im zweiten Schritt [Schritt b)] des erfindungsgemäßen Verfahrens werden die bereitete Antimonpentahalogenid-Vormischung und die Restmenge der zu alkoxylierenden Verbindung zusammengebracht. Das Zusammenbringen erfolgt vorzugsweise unter Rühren, um eine gute Vermischung zu erreichen. Die Mischtemperatur kann innerhalb weiter Grenzen variieren, sie liegt vorzugsweise bei 20 bis 80 °C. Die beiden Teile können so vereinigt werden, daß die Katalysator-Vormischung zur Restmenge hinzugefügt wird (portionsweise oder kontinuierlich) oder die Restmenge zur Katalysator-Vormischung. Die so erhaltene Mischung (Lösung) stellt die zu alkoxylierende Startmischung dar. Wie bereits erwähnt kann im Schritt b) die gesamte bereitete Katalysator- Vormischung oder nur ein Teil davon eingesetzt werden, wobei in jedem Fall darauf zu achten ist, daß in der Startmischung auch eine wirksame Katalysatormenge vorliegt, das heißt eine Antimonpentahalogenid-Menge von mindestens 0,05 Mol-%, vorzugsweise von mindestens 0,2 Mol-%, Molprozente bezogen auf die Molmenge der zu alkoxylierenden Verbindung. Mit weniger als 0,05 Mol-% verläuft die Alkoxylierungsreaktion bereits sehr langsam, und mit mehr als 1 Mol-% kann bereits Farb- und Nebenproduktbildung auftreten. Die Katalysatormenge liegt also im Bereich von 0,05 bis 1 Mol-%, vorzugsweise 0,2 bis 0,6 Mol-%, Molprozente bezogen auf die Molmenge an zu alkoxylierender Verbindung.

Im dritten Schritt [Schritt c)] des erfindungsgemäßen Verfahrens wird die erhaltene Startmischung in üblicher Weise alkoxyliert, das heißt mit dem gewählten Alkylenoxid umgesetzt. Die Alkoxylierung wird man also vorzugsweise derart durchführen, daß man die Startmischung unter Rühren auf eine Temperatur von 40 bis 160 °C bringt, vorzugsweise 60 bis 140 °C, und das Alkylenoxid unter Einhaltung der genannten Temperatur portionsweise oder kontinuierlich zudosiert. Während der Umsetzung kann Normaldruck oder ein erhöhter Druck vorliegen. In der Regel wird die Alkoxylierung bei einem Druck von 2 bis 6 bar durchgeführt. Nach Beendigung der Alkylenoxidzugabe wird die Mischung noch einige Zeit bei der genannten Temperatur zur Nachreaktion gehalten, wobei der Druck immer mehr absinkt und das Ende der Umsetzung anzeigt. Die Menge an Alkylenoxid richtet sich nach dem Zweck, für den das Alkoxylat eingesetzt wird und liegt im allgemeinen bei 1 bis 30 mol, vorzugsweise 2 bis 15 mol und insbesondere 2 bis 8 mol, pro mol zu alkoxylierender Verbindung. Das erhaltene Umsetzungprodukt kann oft schon als solches, das heißt ohne Abtrennung des Katalysators, eingesetzt werden.

Im Falle, daß ein katalysatorfreies Alkoxylat gewonnen werden soll, kann man das Umsetzungsprodukt zum Beispiel mit Wasser oder Lauge zur Zerstörung des Katalysators versetzen, worauf die organische Phase (das ist das angestrebte Alkoxylat) von der wäßrigen Phase abgetrennt wird.

Das erfindungsgemäße Verfahren liefert Alkoxylate in hoher Ausbeute, die nicht nur eine enge Homologenverteilung, sondern auch eine gute Farbqualität aufweisen. Sie können deshalb auch dort zum Einsatz kommen, wo Farbqualität besonders wichtig ist. Das erfindungsgemäße Verfahren bietet ferner aufgrund der bereiteten, relativ konzentrierten Katalysator-Vormischung eine einfache und problemlosere Handhabung von Antimonpentahalogenid bezüglich seines Einsatzes und seiner Dosierung bei technischen Alkoxylierungsprozessen. Die beim erfindungsgemäßen Verfahren bereitete, konzentrierte Katalysator-Vormischung ist bei Raumtemperatur oder darunter nicht rauchend und längere Zeit ohne Aktivitätsverluste haltbar, so daß sie auch für mehrere Alkoxylierungen verwendet werden kann.

Wenn auch die Art der beim erfindungsgemäßen Verfahren vorliegenden Komponenten, Alkylenoxid, Antimonpentahalogenid und aktive H-Atome enthaltende Verbindung, nicht kritisch ist, sei dazu folgendes ausgeführt:

Als Alkylenoxide werden vorzugsweise Ethylenoxid, Propylenoxid und/oder Butylenoxid eingesetzt, wobei Ethylenoxid und/oder Propylenoxid bevorzugt ist. Besonders bevorzugt ist Ethylenoxid.

Als Antimonpentahalogenid wird vorzugsweise Antimonpentabromid oder Antimonpentachlorid eingesetzt, wobei letzteres bevorzugt ist.

Als aktive H-Atome enthaltende Verbindungen eignen sich nicht nur Hydroxylgruppen enthaltende Verbindungen, sondern auch Amine, Amide, Säuren, Phenole, Kresole und dergleichen, wobei jedoch die ersteren bevorzugt sind. Von den Hydroxylgruppen enthaltenden Verbindungen seien die Alkohole, Aminoalkohole, Glykole, Glykolmonoether, Glycerin und dergleichen genannt, wobei die Alkohole bevorzugt sind. Sie können aus nativer Quelle oder aus Synthese-Prozessen stammen, vor allem primär oder sekundär, linear oder verzweigt, ein- oder mehrwertig sein, zum Beispiel Oxo-Alkohole, Guerbet-Alkohole, Ziegler-Alkohole, Fettalkohole und dergleichen. Bevorzugte Alkohole sind die primären und geradkettigen oder verzweigten $C_3$ bis $C_{24}$-Alkanole, vorzugsweise $C_6$ bis $C_{18}$-Alkanole. Als Beispiele für die bevorzugten Alkohole seien genannt: Butanol, Amylalkohol, Hexanol, Nonanol, Isononylalkohol, Decanol, Laurylalkohol, Isotridecylalkohol, Stearylalkohol, Cocosfettalkohol und Gemische davon, wie $C_{12}$ bis $C_{14}$-Alkanolgemische, ferner 2-Ethylhexanol, 2-Hexyldecanol, 2-Octyldecanol und ähnliche Guerbet-Alkohole.

Die Erfindung wird nun an Beispielen noch näher erläutert.

Beispiel 1

731 g (3,75 mol) eines geradkettigen $C_{12}$ bis $C_{14}$-Alkanolgemisches sollten mit 660 g (14,9 mol) Ethylenoxid unter Einsatz von 0,30 Mol-% Antimonpentachlorid als Katalysator, das sind 3,4 g (0,0114 mol) Antimonpentachlorid, alkoxyliert werden.

Zunächst wurde eine Antimonpentachlorid-Vormischung bereitet, wobei die gesamte vorgesehene $SbCl_5$-Menge, das sind die genannten 3,4 g, und 7,3 g (0,037 mol) Alkanolgemisch, das sind 1 Gew.-%, eingesetzt wurden. Die Bereitung der Vormischung unter trockenem Stickstoff als Schutzgas erfolgte in der Weise, daß die 3,4 g $SbCl_5$ kontinuierlich unter Rühren und Einhalten einer Temperatur von 30 bis 50 °C in die 7,3 g n-$C_{12}$ bis $C_{14}$-Alkohol eingebracht wurden. Die erhaltene, an der Luft nicht rauchende Vormischung (10,7 g) hatte eine Konzentration von 30 Mol-% $SbCl_5$, bezogen auf die Molmenge an Alkanol.

Nun wurden die 10,7 g Katalysator-Vormischung, das ist die gesamte bereitete Vormischung, und die restliche Menge an Alkanolgemisch, das sind 723,7 g, unter Rühren bei 70 bis 80 °C vermischt. Die erhaltene Startmischung bestand aus 3,4 g (0,0114 mol) $SbCl_5$ und 731 g (3,75 mol) n-$C_{12}$ bis $C_{14}$-Alkohol und enthielt 0,30 Mol-% $SbCl_5$, bezogen auf die gesamte Alkanolmenge.

Die Ethoxylierung der Startmischung erfolgte in einem 3-Liter-Rührautoklaven. Dazu wurde die Mischung auf 80 °C erhitzt. In die erhitzte Startmischung wurden unter Rühren und bei einer Temperatur von 80 bis 120 °C 660 g gasförmiges Ethylenoxid (15,0 mol) in dem Maße zugeführt, wie es abreagierte. Der Druck im Autoklaven betrug 2 bis 3 bar. Nachdem die genannte Menge Ethylenoxid zudosiert war, wurde die Mischung noch 2 Stunden bei 120 °C zur Nachreaktion gerührt, worauf der Druck bei 0,8 bar konstant blieb und das Ende der Reaktion anzeigte. Der Autoklaveninhalt (das Reaktionsprodukt) wurde zur Zerstörung des Katalysators mit 20 g von einer 10gew.%igen wäßrigen NaOH-Lösung versetzt, die Mischung bei 90 °C 0,5 Stunden gerührt und danach die wäßrige Phase von der organischen Phase abgetrennt.

Das erhaltene $C_{12}$ bis $C_{14}$-Alkanolalkoxylat mit im statistischen Mittel 4 mol Ethylenoxid/Mol und enger Homologenverteilung wurde in praktisch quantitativer Ausbeute erhalten und hatte eine Hazen-(A.P.H.A.)-Farbzahl von 120, gemessen nach DIN 53 409.

Beispiele 2 bis 6

Diese Beispiele sind in der nachstehenden Tabelle zusammengefaßt. Die Tabelle enthält der Vollständigkeit halber auch das Beispiel 1.

Von den in den Beispielen 2 bis 6 zu alkoxylierenden Verbindungen wurde Jeweils eine Katalysator-Vormischung durch Auflösen der angegebenen Gramm $SbCl_5$ in den angegebenen Gramm Alkohol unter Einhaltung der angegebenen Mischtemperatur hergestellt. Mit der erhaltenen Vormischung und dem in Rede stehenden Alkohol wurde die Startmischung durch Mischen der beiden Komponenten wie in Beispiel 1, das heißt bei 70 bis 80 °C, bereitet, wobei in den Beispielen 2 bis 4 die gesamte hergestellte Katalysator-Vormischung eingesetzt wurde und in den Beispielen 5 und 6 nur ein Teil davon. Die Alkoxylierung der Startmischungen wurde ebenfalls wie in Beispiel 1 durchgeführt mit den in der Tabelle angegebenen Bedingungen. Was die Ergebnisse der Beispiele 2 bis 6 betrifft, wurde auch hier eine praktisch quantitative Ausbeute und eine enge Homologenverteilung erhalten, die Farbe der Alkoxylate ist in der Tabelle angegeben.

Vergleichsbeispiel

Beispiel 1 wurde wiederholt, mit dem Unterschied, daß das $SbCl_5$ nicht in Form der beschriebenen Vormischung, sondern als solches eingesetzt wurde. Es wurde also in der Weise vorgegangen, daß die 731 g (3,75 mol) des $C_{12}$ bis $C_{14}$-Alkanolgemisches in dem 3-Liter-Rührautoklaven vorgelegt und die 3,4 g (0,0114 mol) Antimonpentachlorid in den vorgelegten und auf Raumtemperatur befindlichen Alkohol unter Rühren eingebracht wurden, worauf die Mischung, wie in Beispiel 1, mit den 660 g (15,0 mol) Ethylenoxid ethoxyliert wurde.

Das erhaltene $C_{12}$ bis $C_{14}$-Alkanolalkoxylat mit im statistischen Mittel 4 mol Ethylenoxid/Mol zeigte ebenfalls die für $SbCl_5$-Katalyse typische enge Homologenverteilung. Seine Hazen-Farbzahl war 300, das heißt dieses Alkoxylat war deutlich stärker gefärbt als die Alkoxylate der erfindungsgemäßen Beispiele.

## TABELLE

| Bei-spiel Nr. | zu alkoxylierende Verbindung | Schritt a) | | $SbCl_5$-Gehalt in der Katalysator-Vormischung (Mol-%) | Temperatur bei der Herstellung der Vormischung (°C) |
| --- | --- | --- | --- | --- | --- |
| | | Katalysator-Vormischung bestehend aus | | | |
| | | Alkohol (g) | $SbCl_5$ (g) | | |
| 1 | n-$C_{12}$ bis $C_{14}$-Alkohol | 7,3 | 3,4 | 30 | 30 bis 50 |
| 2 | 2-Ethylhexanol | 22,7 | 5,3 | 10 | 30 bis 40 |
| 3 | n/iso-$C_{11}$-Alkohol | 72,0 | 6,0 | 5 | 20 bis 40 |
| 4 | n-$C_{18}$-Alkohol | 13,9 | 3,1 | 20 | 50 bis 60 |
| 5 | $C_{16}$ bis $C_{20}$-Guerbet-Alkohol | 250,0 | 20,0 | 8 | 20 bis 40 |
| 6 | iso-$C_{13}$-Alkohol | 82,7 | 20,0 | 16 | 30 bis 50 |

(Fortsetzung)

**TABELLE** (Fortsetzung)

| Schritt b) — Startmischung bestehend aus | | | Schritt c) | Alkoxylat |
| Katalysator-Vormischung (g) | Alkohol (g) | SbCl5-Gehalt in der Startmischung (Mol-%) | Ethylenoxid-Menge in mol pro mol Alkohol | Farbzahl (Hazen) |
|---|---|---|---|---|
| 10,7 | 724 | 0,30 | 4 | 120 |
| 28,0 | 620 | 0,36 | 6 | 150 |
| 78,0 | 800 | 0,40 | 7 | 120 |
| 17,0 | 1 336 | 0,21 | 2 | 150 |
| 29,0 | 650 | 0,30 | 3 | 180 |
| 38,0 | 790 | 0,60 | 8 | 140 |

**Patentansprüche**

1. Verfahren zur Herstellung von Alkoxylaten mit einer engen Homolgenverteilung durch Umsetzung von mindestens ein aktives H-Atom enthaltenden Verbindungen mit einem Alkylenoxid unter Verwendung von Antimonpentahalogeniden als Katalysator, dadurch gekennzeichnet, daß

a) mit einer Teilmenge der zur Alkoxylierung vorgesehenen Verbindung eine maximal 30 Mol-%, bezogen auf die eingesetzte Verbindungsmenge, Antimonpentahalogenid enthaltende Katalysator-Vormischung bereitet wird, durch Auflösen von Antimonpentahalogenid in der eingesetzten Verbindungsmenge unter Einhaltung einer Temperatur von maximal 60 °C und der genannten Konzentration an Antimonpentahalogenid von maximal 30 Mol-%, und

b) diese Vormischung als Katalysatorkomponente eingesetzt wird, indem man die gesamte bereitete Vormischung oder einen Teil davon mit der restlichen Menge der zu alkoxylierenden Verbindung vereinigt, und

c) die so erhaltene Startmischung mit dem Alkylenoxid umgesetzt wird, mit der Maßgabe, daß in der Startmischung eine Antimonpentahalogenidmenge von 0,05 bis 1 Mol-% vorliegt, bezogen auf die zu alkoxylierende Verbindungsmenge.

2. Verfahren nach Anspruch 1, dadurch gekennzeichnet, daß eine 5 bis 30 Mol-% Antimonpentahalogenid enthaltende Katalysator-Vormischung bereitet wird.

3. Verfahren nach Anspruch 1, dadurch gekennzeichnet, daß eine 10 bis 20 Mol-% Antimonpentahalogenid enthaltende Katalysator-Vormischung bereitet wird.

4. Verfahren nach einem der Ansprüche 1 bis 3, dadurch gekennzeichnet, daß beim Auflösen des Antimonpentahalogenids eine Temperatur von 20 bis 60 °C eingehalten wird.

5. Verfahren nach einem oder mehreren der Ansprüche 1 bis 4, dadurch gekennzeichnet, daß in der Startmischung eine Antimonpentahalogenidmenge von 0,2 bis 0,6 Mol-% vorliegt.

6. Verfahren nach einem oder mehreren der Ansprüche 1 bis 5, dadurch gekennzeichnet, daß Antimonpentachlorid als Katalysator, Ethylenoxid oder Propylenoxid als Alkylenoxid und geradkettige oder verzweigte primäre $C_6$ bis $C_{18}$-Alkanole als aktive H-Atome enthaltende Verbindungen eingesetzt werden.

Europäisches
Patentamt

**EUROPÄISCHER
RECHERCHENBERICHT**

Nummer der Anmeldung

**EP 91 10 6266**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | JOURNAL OF THE AMERICAN CHEMISTS' SOCIETY, Band 58, Nr. 5, Mai 1981, Seiten 650-655; L. FARKAS et al.: "Determination of molar mass distribution of alkyl polyethylene glycol ethers" <br> * Seite 650, Spalte 2, letzter Abschnitt - Seite 651, Spalte 1, Abschnitt 2; Seite 651, Tabelle 1 * <br> - - - | 1,5-6 | C 07 B 41/04 <br> C 07 C 43/11 <br> C 07 C 41/03 |
| D,A | GB-A-7 965 08  (HENKEL) <br> * Beispiele 1-6; Ansprüche * <br> - - - | 1-6 | |
| D,A | US-A-3 359 331  (S.B. BAKER et al.) <br> * Spale 3, Zeile 65 - Spalte 4, Zeile 21; Ansprüche * <br> - - - - - | 1-6 | |

RECHERCHIERTE
SACHGEBIETE (Int. Cl.5)

C 07 B 41/00
C 07 C 41/00
C 07 C 43/00

Der vorliegende Recherchenbericht wurde für alle Patentansprüche erstellt

| Recherchenort | Abschlußdatum der Recherche | Prüfer |
|---|---|---|
| Den Haag | 02 Juli 91 | ZERVAS B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
------------------------------------------
& : Mitglied der gleichen Patentfamilie, übereinstimmendes Dokument